(19) Europäisches Patentamt
European Patent Office
Office européen des brevets

(11) Publication number: 0 315 100 A2

# (12) EUROPEAN PATENT APPLICATION

(21) Application number: 88118112.7

(51) Int. Cl.4: C07C 63/38 , C07C 51/265

(22) Date of filing: 31.10.88

(30) Priority: 02.11.87 JP 275693/87

(43) Date of publication of application: 10.05.89 Bulletin 89/19

(84) Designated Contracting States: DE FR GB

(71) Applicant: Teijin Petrochemical Industries Ltd.
1-1, 2-chome, Uchisaiwai-cho
Chiyoda-ku Tokyo(JP)

(72) Inventor: Hirose, Isao
2-2-27, Matsunami
Chigasaki-shi Kanagawa-ken(JP)

(74) Representative: Kraus, Walter, Dr. et al
Patentanwälte Kraus, Weisert & Partner
Thomas-Wimmer-Ring 15
D-8000 München 22(DE)

## (54) Process for producing 2,6-naphthalenedicarboxylic acid from 2,6-diisopropylnaphthalene.

(57) A process for producing 2,6-naphthalenedicarboxylic acid (NDA) which comprises oxidizing 2,6-diisopropylnaphthalene (DIPN) or its oxidation derivative with a gas containing molecular oxygen in a solvent containing at least 70 % by weight of at least one aliphatic monocarboxylic acid selected from the group consisting of acetic acid and propionic acid; wherein the oxidation is carried out

(a) using at least 1.5 parts by weight per part by weight of the entire reaction mixture composed of the material to be oxidized, its intermediates and product from these which are present in the oxidastion reaction system,

(b) using a catalyst comprising manganese, or a heavy metal mixture composed of cobalt and manganese with a manganese content of not more than 20 atomic percent, bromium and potassium in an amount satisfying the following expressions (i) to (v) simultaneously

$$\text{(i)} \quad (X + Y) \geq (100/Z + Z/20)$$

$$\text{(ii)} \quad (X + Y) \leq 50$$

$$\text{(iii)} \quad Z \leq 60$$

$$\text{(iv)} \quad Z < W \leq 8Z$$

$$\text{(v)} \quad W \leq 120$$

wherein X, Y, Z and W are the gram atoms, in atomic percent, of cobalt, manganese, bromine and potassium,
respectively, per mole of the starting 2,6-diisopropylnaphthalene or its oxidation derivative, and

(c) at a temperature in the range of 180 to 220 °C.

EP 0 315 100 A2

This invention relates to a process for producing 2,6-naphthalenedicarboxylic acid (sometimes "NDA" for short) by oxidizing 2,6-diisopropylnaphthalene (sometimes "DIPN" for short) or its oxidation derivative. More specifically, this invention relates to a process for producing NDA in a very high yield by oxidizing DIPN in a solvent containing acetic acid and/or propionic acid in the presence of a catalyst comprising a heavy metal composed of cobalt and/or manganese, bromine and potassium.

2,6-Naphthalenedicarboxylic acid (NDA) and its derivatives such as its esters and acid chloride are valuable as a dibasic acid component of various polyesters and polyamides. Particularly, polyethylene naphthalate formed from NDA and ethylene glycol has higher thermal resistance and mechanical properties than polyethylene terephthalate and are useful for production of films or fibrous products of high quality and performance.

For the production of NDA, oxidation of 2,6-dimethylnaphthalene has been known. For example, 2,6-dimethylnaphthalene is catalytically oxidized with molecular oxygen in an acetic acid solvent in the presence of a catalyst composed of cobalt, manganese and bromine. In this process, the oxidation itself of 2,6-dimetylnaphthalene to NDA is relatively easy and the desired NDA of relatively high purity can be obtained in a relatively high yield. However, a method of producing 2,6-dimethylnaphthalene as the starting material is complex, and it is difficult to obtain in large quantities at low costs. Methylation of naphthalene, isomerization of dimethylnaphthalene, disproportionation of monomethylnaphthalene, and transalkylation of these are known as a method of synthesizing 2,6-dimethyl naphthalene. These methods cannot avoid formation of isomers other than 2,6-dimethylnaphthalene, particularly 2,7-dimethylnaphthalene, and it is extremely difficult to separate the 2,6-isomer from the mixed dimethylnaphthalenes because the 2,6-isomer is very close or similar to the 2,7-isomer in melting point, boiling point and dissolution characteristics.

On the other hand, a mixture of diisopropylnaphthalene isomers having a high proportion of the 2,6-isomer can be easily synthesized from naphthalene and propylene, and the separation of the 2,6-isomer from the diisopropylnaphthalene isomeric mixture is comparatively easily.

The investigations of the present inventor, however, showed that when DIPN is oxidized in accordance with the known methods, the yield of NDA is as low as below 50 % under reaction conditions which are suitable for oxidation of p-xylene or 2,6-dimethylnaphthalene, and that because large amounts of by-products are formed, the purity of NDA is also low. Accordingly, it is virtually impossible to obtain NDA from DIPN industrially by these known methods, and the production of NDA from DIPN by these methods has been disregarded industrially.

No clear reason has been assigned to the fact that the oxidation of DIPN does not give satisfactory results. The present inventor, however, theorizes as follows on the basis of many experiments he has conducted. When the yield of the desired product NDA is low in this oxidation reaction, the relative yield of trimellitic acid (sometimes "TMA" for short), which is a by-product resulting from cleavage of the naphthalene nucleus, is high. In an extreme case, a tarry or resinous polycondensate of an unknown structure is formed in large amounts. In the oxidation of DIPN having an isopropyl group and a naphthalene nucleus which have high activity and low oxidation stability unlike the oxidation of other alkyl-substituted aromatic hydrocarbons such as p-xylene or dimethylnaphthalene, radicals and hydroperoxide form very easily and rapidly by hydrogen extraction of the isopropyl group in the early stage of the reaction. However, these radicals and hydroperoxide have low stability to the atmosphere, and their decomposition rapidly induces the formation of a phenolic compound (particularly naphthol) having high oxidation inhibiting tendency and condensability. The decomposition of the phenolic compound subsequently forms a nucleus-cleaved by-product. Hence, the desired oxidation does not sufficiently proceed, and rather side-reactions are promoted.

Previously, the present inventor discovered a process in which NDA is obtained in a high yield while inhibiting the aforesaid side-reactions by oxidizing DIPN or its oxidation derivative using much larger amounts of cobalt and/or manganese based on the material to be oxidized than those known before (see U. S. Patent No. 4,709,088 and European Patent No. 0142719).

The present inventor also developed a new process for producing NDA in which by oxidizing DIPN or its oxidation derivative in the presence of an alkali metal in an excessive amount with respect to bromine used together with cobalt and/or manganese as a catalyst, results equivalent to, or better than, those obtained in the previously proposed process can be obtained without using large amounts of cobalt and/or mananese in the previously proposed process; and also a process in which by replacing part or the whole of the acetic acid solvent with propionic acid in addition to using the alkali metal in the above process, better results than the above process can be obtained (see U. S. Patent No. 4,716,245 and European Patent No. 0204119).

These processes are very useful industrially because they give NDA of higher purity in higher yields

than any of the processes previously known.

Since, however, these processes have been independently developed by the present inventor, the optimal conditions of each of these techniques used singly do not necessarily coincide with those of a combination of two or more of these techniques. Furthermore, it is impossible to anticipate changes in conditions which have to be made when these techniques are combined. This has limited the use of these techniques in combination.

The present inventor continued research work in an attempt to develop an industrially more advantageous process for oxidizing DIPN or its oxidation derivative by using a combination of the techniques in the processes previously proposed by the present inventor and eliminating the aforeaid difficulties while retaining the advantages of the individual techniques. The work has led to the discovery that when a combination of these techniques is employed, a set of optimal conditions which lead to technically and economically optimal results can be obtained by maintaining the atomic ratio of the heavy metals composed of cobalt and/or manganese to bromine in the oxidation catalyst at a specific reciprocal relationship and combining the oxidation conditions of these techniques properly as described below.

According to this invention, there is provided a process for producing 2,6-naphthalenedicarboxylic acid (NDA) which comprises oxidizing 2,6-diisopropylnaphthalene (DIPN) or its oxidation derivative with a gas containing molecular oxygen in a solvent containing at least 70 % by weight of at least one aliphatic monocarboxylic acid selected from the group consisting of acetic acid and propionic acid; wherein the oxidation is carried out

(a) using at least 1.5 parts by weight per part by weight of the entire reaction mixture composed of the material to be oxidized, its intermediates and product from these which are present in the oxidastion reaction system,

(b) using a catalyst comprising manganese, or a heavy metal mixture composed of cobalt and manganese with a manganese content of not more than 20 atomic percent, bromium and potassium in an amount satisfying the following expressions (i) to (v) simultaneously

(i) $(X + Y) \geq (100/Z + Z/20)$

(ii) $(X + Y) \leq 50$

(iii) $Z \leq 60$

(iv) $Z < W \leq 8Z$

(v) $W \leq 120$

wherein X, Y, Z and W are the gram atoms, in atomic percent, of cobalt, manganese, bromine and potassium, respectively, per mole of the starting 2,6-diisopropylnaphthalene or its oxidation derivative, and

(c) at a temperature in the range of 180 to 220 °C.

The present invention provides a new technique of combining the techniques of the aforesaid prior inventions made by the present inventor, which produces unexpected results.

A first feature of the present invention is that the atomic ratio of heavy metals composed of cobalt (X) and/or manganese (Y) to bromine (Z) in the oxidation catalyst is maintained in a specific reciprocal relationship, $XY = \alpha$ or larger. In other words, the amount of the catalytic heavy metals is inversely proportional to the amount of bromine, and when the heavy metals are used in a large amount as the oxidation catalyst, the amount of bromine (Z) used may be small. Conversely, even in the presence of small amounts of heavy metals (X + Y), the use of a relatively large amount of bromine (Z) permits the oxidation reaction to proceed easily to give the desired NDA of high purity in very high yields.

A second feature of the present ivnention is that this reciprocal relationship between the catalytic heavy metals (X + Y) and bromine (Z) is effective only when the atomic ratio of potassium (W) to bromine (Z) is within a specific range.

It is well known that generally in the oxidation of an alkyl-substituted aromatic hydrocarbon, particularly p-xylene or 2,6-dimethylnaphthalene, with molecular oxygen in an aliphatic monocarboxylic acid using a catalyst comprising heavy metals such as cobalt and/or manganese and bromine, there is an optimal range in the amount of the catalyst used in this reaction. It is also known to carry out this reaction in the presence of an alkali metal such s sodium or potassium, or ammonium.

So far, however, only the proportional relationship $X = \alpha Y$ has been known to exist between the amount of catalytic heavy metals and the amount of bromine. Namely, it has been known that when the catalytic

heavy metals are used in large amounts, bromine is used correspondingly in a large amount. The reciprocal relation XY = α as in the process of this invention has been known. Moreover, it has been quite unknown prior to the present invention that this reciprocal relationship is effective only when potassium (not another alkali metal) exists in the reaction system, and the ratio of potassium to bromine is within the specfic range. This fact is therefore peculiar to the oxidation of DIPN and cannot be at all anticipated from the information obtained on the oxidation of other general alkyl-substituted aromatic hydrocarbons.

The process of this invention will be described in detail.

The starting material used in the process of this invention is 2,6-diisopropylnaphthalene (DIPN) or its oxidation derivative or a mixture thereof. They are preferably, but not necessary, of high purity. It may contain other components so long as they are permissible in regards to effects on the oxidation reaction or to the purity or coloration of the resulting NDA.

The oxidation derivative of DIPN denotes a compound which forms as a result of oxidation of DIPN and is oxidized in the reaction system to give the final desired NDA. Specifically, the starting material of this invention is represented by the following general formula (1)

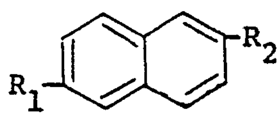 ..... (1)

wherein $R_1$ is a group selected from the class consisting of

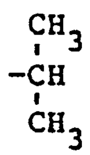 , 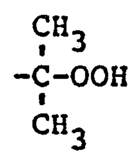 and 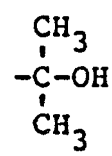,

and $R_2$ is a group selected from the class consisting of the groups R, and

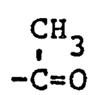,

-COOH and -CHO, and may be idetntical with or different from $R_1$.

Compounds of formula (1) in which $R_1$ and $R_2$ are identical or different and are selected from

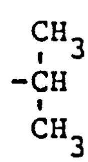

and

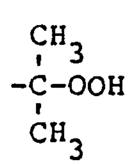

are preferred as the starting material for use in the process of this invention.

The oxidation catalyst used in the process of this invention comprises

(1) manganese or a mixture of cobalt and manganese (component A),

(2) bromine (component B), and

(3) potassium (component C).

The catalyst may be in any form which can be dissolved in the oxidation reaction system of ths invention.

Sources of cobalt and manganese forming component A include, for example, inorganic salts such as oxides, hydroxides, carbonates, and halides (particularly bromides) of cobalt and manganese, and organic acid salts such as aliphatic monocarboxylic acid salts such as acetic acid and propionic acid salts and aromatic carboxylic acid (particularly, NDA) salts of cobalt and manganese. The bromides and the aliphatic monocarboxylic acid salts (particularly, acetates and propionates) are preferred.

Sources of bromine forming component B may be any organic or inorgnic compounds which dissove in the oxidation reaction system to produce a Br ion. Specific examples are molecular bromine, inorganic bromine compounds such as hydrobromic acid and hydrobromide, and organic bromine compounds, for example, alkyl bromides such as methyl bromide and ethyl bromide, bromoform, ethylene bromide, and brominated fatty acids such as bromoacetic acid and polybromoacetic acid. Of these, molecular bromine, hydrogen bromide, potassium bromide, cobalt bromide and manganese bromide are preferred.

It is considered that in the oxidation catalyst, component B is coordinated with, or bonded to, component A, or components A and B form a counter ion, as ions of single salts or complex salts, and are par ticipated in the reaction. Accordingly, metal elements and insoluble metal compounds which do not easily form such ions during the reaction, or organic bromine compounds which do not decompose at the reaction temperature and do not easily liberate a bromine ion, such as nuclearly brominated aromatic compounds, have little or no catalytic effects.

Bromine added to the oxidation reaction system in this invention, in whatever form it may be, is liable to add partly to the isopropyl side-chain of DIPN or its oxidation derivative either directly or secondarily and to form a side-chain brominated derivative of this starting material. The side-chain brominated derivative is decomposed more or less under the oxidation reaction conditions in the present invention to release a bromine ion. Accoridngly, the side-chain brominated derivative of the starting material is also effective as a source of catalyst component B in the process of this invention.

Potassium, component C, used in the process of this invention may be of any type which can dissolve in the oxidation reaction system. Specifically, potassium hydroxide, potassium carbonate, potassium acetate, potassium propionate and potassium bromide are preferred. Use of inorganic strong acid salts of potassium such as potassium sulfate, nitrate and chloride (exluding the bromide) should be avoided.

Another alkali metal such as sodium and lithium is less effective than potassium as component C. But potassium may be used in combination with a minor proportion of the other alkali metal. The use of ammonium or an alkaline earth metal such as calcium or barium as component C in place of potassium is hardly effective for practical purposes. But the combined use of potassium with such materials is not particularly detrimental.

Components A, B and C used in the process of this invention promote the reactions as ions of cobalt, manganese, bromine and potassium constituting these components. The counterions forming the compounds which produce these ions have subsidiary effects such as the ability to retain the solubility and dispersibility of these compounds, but are essentially ineffective in promoting the reaction of this invention.

Preferably, the components A, B and C are used in the form of salts of the ions of cobalt, manganese, bromine and potassium constituting these compounds with each other or with the acetic acid or propionic acid used as solvent in the reaction. Other salts which bring unwanted other ions to the reaction system should better be not used in this invention unless they are easily volatile and non-accumulating. Exceptions are "salts which under conditions of use, form only compounds which are non-toxic and are not accumulated within the system, such as carbon dioxide gas and water which are the by-products of the present invention", for example carbonates, hydroxides or free acids.

As the present inventor describes in U. S. Patent No. 4,709,088 (European Patent No. 0142719), the proportion of component A used relative to the starting material and the concentration of component A relative to the solvent should better be as high as possible as far as the reaction yield in the oxidation reaction is concerned. Thus, the upper limits to these are difficult to determine in practice. Use of an excessive amount of the catalyst is industrially undesirable because it will reduce productivity or degrade the quality of the final product. If the oxidation is carried out under the combined conditions shown in this invention, the use of a much smaller amount of the catalyst than those described in the above patent can give highly pure NDA in a high yield.

As stated above, the amount of component A of the catalyst is inversely proportional to the amount of component B, and the amount of component B is proportional to the amount of component C.

Let the gram-atoms in perecent of cobalt, manganese, bromine and potassium constituting these components per mole of the starting DIPN or its oxidation derivative be X, Y, Z and W, the total amount of cobalt and manganese should be within the following lower limit formula

(i) $(X + Y) \geqq (100/Z + Z/20)$

preferably

$(X + Y) \geqq (160/Z + Z/8)$

and its upper limit formula

(i) $(X + Y) \leqq 50$

preferably

$(X + Y) \leqq 40$

The lower limit of (X+Y) will be explained by citng specific numerical values. For example, if the amount of bromine (Z) is as small as 0.5 atomic percent, relatively large amounts of cobalt and manganese are required as shown by $(X+Y) \geqq 20.25$ atomic percent, preferably $(X+Y) \geqq 32.63$ atomic percent. In contrast, if $Z=20$ atomic percent, $(X+Y) \geqq 6$ atomic percent, preferrably $(X+Y) \geqq 10.5$ atomic percent. If bromine is used in a large amount of 40 atomic percent, $(X+Y) \geqq 4.5$ atomic percent, preferably $(X+Y) \geqq 9$ atomic percent, thus requiring small amounts of cobalt and manganese. The lower limit of (X+Y) does not fall below 0.5 atomic percent, preferably 9 atomic percent, as can bed seen from the above formulae. Thus, this is the lower limit of the total amount of cobalt and manganese used.

Likewise, the lower limit of the amount of bromine used is 2.5 atomic percent, preferably 4 atomic percent where the total amount of X + Y is 50 atomic percent, preferably 40 atomic percent, which is an upper limit.

If the total amount of cobalt and manganese or the amount of bromine is lower than the above lower limit formula, the yield of the desired NDA is lowered, and the formation of by-products increases, and the decrease of the yield is increases in proportion to the extent to which the above amount decreases from the lower limit. On the other hand, if one or both of the sum of cobalt and manganese and bromine exceed the upper limits, the yield of NDA evidently decreases, but the extent of decrease is less than when the amount is less than the lower limits.

In the process of this invention, manganese alone or a mixture of cobalt and manganese with a manganse content of 20 to 100 atomic percent is used. Within this range, variations in the yield of NDA owing to the mixing ratio of cobalt and manganese are much less than the variations in yield owing to the aforesaid amounts of cobalt and manganese or bromine. The best mixing ratio is within a manganese content of 30 to 70 atomic percent from the viewpoint of the yield and purity of NDA that can be technically attained. In view of economical considerations involving the cost of the catalyst and expenditures, a cobalt/manganese mixture having a high manganese content or manganese alone is sometimes better.

Use of cobalt alone or a cobalt/manganese mixture with a manganese content of less than 20 atomic percent is undesirable because it is apparently less effective.

The amount of bromine constituting component B used in the process of this invention not only depends upon the amount of component A used, but also is proportional to the amount of potassium used as component C as shown by the following expressions.

(iv) $Z<W \leqq 8Z$ (preferably 6Z)

Components B and C should be used in amounts which do not exceed the ranges defined by the following upper limit formulae.

(iii) $Z \leqq 60$ (preferably 50)

(v) $W \leqq 120$ (preferably 100)

(The units are all atomic percent.)

Investigations of the present inventor have shown that the bromine/potassium atomic ratio is best in the reaction of this invention when it is about 1:2 (i.e., W = about 2Z). The yield of NDA decreases, although gradually, as this ratio is away from the optimal value. When this atomic ratio is lsss than 1:1, namely $W \leqq Z$, or exceeds 1:8, namely $W<8Z$, the proceeding of the reaction rapidly becomes difficult, and the yield of NDA decreases markedly.

If the ratio of bromine to potassium is within the range specified in this invention, the decrease of the yield of NDA is comparatively slight even if their amounts Z and W exceed the upper limit formulae (iii) and (v). However, to use large amounts of components B and/or C at the sacrifice of economical burdens brings about no advantage.

The reaction solvent used in the process of this invention contains at least 70 % by weight, preferably at least 80 % by weight, of acetic acid or propionic acid. The remainder of the solvent is not particularly

limited so long as it does not substantially affect the oxidation reaction of this invention adversely. For example, it may be water or another aliphatic monocarboxylic acid such as n-butyric acid, or another organic compound. Water forms as a by-product in the reaction of this invention, and the presence of water in the reaction solvent during the reaction is virtually difficult to avoid completely. The presence of water in the oxidation of DIPN tends to adversely affect the reaction to a greater extent than in the oxidation of p-xylene or dimethylnaphthalene. When the reaction solvent used in this invention contains water, its content should desirably be not more than 30 % by weight, preferably not more than 20 % by weight, especially preferably not more than 10 % by weight.

Propionic acid is better than acetic acid as the reaction solvent used in this invention from the viewpoint of the yield and purity of the desired NDA. Acetic acid, however, is lower in cost. To set a compromise between the two, it is convenient to use a mixture of acetic acid and propionic acid making use of their respective advantages. The mixed solvent does not vary in effects on the reaction according to the mixing ratio between acetic acid and propionic acid. If the proportion of propionic acid is too low, the effect of propionic acid to increase the yield cannot be expected. Advantageously, the proportion of propionic acid is at least 25 % by weight, preferably at least 33 % by weight, based on the total weight of the mixture. If the proportion of propionic acid is higher, the difference in effect according to the ratio of the two is relatively small or is hardly noticed, and the yield of NDA is nearly equal to that obtained when propionic acid is used alone.

The solvent used in the process of this invention serves to dissolve the starting material, the catalyst and molecular oxygen at least partly and aid in contacting of these materials. In addition, it promotes or facilitates dispersion or removal of heat, flowing of the reaction system, and crystal growth of the product, etc., and permit easy industrial practice of the process of this invention. The amount of the solvent should therefore be determined according to these purposes, and is not essentially limited. In practice, to permit the reaction to proceed smoothly and achieve the objects of this invention fully, the solvent should be used in an amount of at least 1.5 parts by weight, preferably 2 parts by weight, per part·by weight of the total reaction mixture comprising the starting material, its intermediates and oxidation products from them. Use of excessively large amounts of the solvent, however does not promote the reaction, but results in increasess losses of the solvent owing to oxidation and combustion. For practical purposes, it is advantageous to use the solvent in an amount of not more than 10 parts by weight, preferably not more than 5 parts by weight

It has previously been known that generally in the liquid-phase oxidation of alkylnaphthalenes, the reaction yield can be increased by decreasing the concentration of the starting material in the reaction system over that in the oxidation of alkylbenzenes such as p-xylene. In the present invention, too, if the concentration of DIPN or its oxidation derivative as the starting material is high in the reaction system during the reaction, its oxidtion in the initial stage proceeds too rapidly and side reactions are promoted. Consequently, the yield of NDA decreases markedly. Accordingly, it is necessary to maintain the concentration of the starting material in the reaction system as low as possible so that such a rapid proceeding of oxidation in the initial stage may be prevented.

In view of the reaction yield, it is not desirable to perform the oxidation reaction of this invention by a batchwise reaction procedure or a similar procedure by which the concentration of the starting material in the initial stage becomes very high. The results of the present inventor's investigations show that to maintain the concentration of the starting DIPN or its oxidation derivative in the reaction system low within the conditions speicifed in the invention and thereby to obtain NDA of high purity in high yields, it is desirable to perform the oxidation reaction while adding the starting material continuously or intermittently at a rate of not more than 1 part by weight per 2 parts by weight of the solvent per hour. The addition of the starting material is preferably performed continuously. If it has to be effected intermittently, it is desirable to add the starting material portionwise many times, preferably more than 10 times per hour. each portion consisting of the smallest possible amount of the starting material.

In the process of this invention, pure oxygen or a gaseous mixture obtained by diluting pure oxygen with an inert gas. For practical purposes, air is most easily available molecular oxygen-containing gas. Air may be used directly, or as required, after concentrating or diluting it with oxygen or an inert gas.

The oxidation reaction in the process of this invention may be carried out under normal atmospheric pressure, but proceeds more rapidly under elevated pressure. The reaction generally proceeds more rapidly as the partial oxygen pressure in the system is higher. For practical purposes, the sufficient partial oxygen pressure is at least 0.1 $kg/cm^2$.abs., preferably at least 0.2 $kg/cm^2$.abs. to 8 $kg/cm^2$.abs. When oxygen is used as a mixture with an inert gas, the reaction proceeds rapidly under a total pressure of not more than 30 $kg/cm^2$-G to give NDA in a high yield.

The reaction proceeds even at 60 °C. But the rate of the reaction at this temperature is slow and not practical. If the reaction temperature exceeds 240 °C, the proportion of by-products formed increases and

the yield of NDA decreases. At high temperatures, the loss of the solvent by combustion is not negligible. The reaction temperature most suitable for the oxidation in accordance with this invention 200 ± 10 °C. But to have the effect of the process of this invention exhibited to a maximum for practical purposes, the reaction should be carried out at a temperature at least within the range of 180 to 220 °C. To increase the effect of the process of this invention further, it is sometimes effective to adjust the reaction temperature within this temperature range properly according to the other conditions. For example, when the manganese/cobalt ratio is high, the reaction temperature is set at a slightly higher point, and when the oxidation is carried out under a relatively low elevated pressure, the temperature is set at a slightly lower point.

In performing the oxidation reaction in accordance with the process of this invention, the catalyst, the solvent and the starting material are fed into a reactor simultaneously or separately (after optionally heating them). The gas containing molecular oxygen is blown into the reactor, and while maintaining the reaction mixture at a predetermined pressure and temperature, the reaction is carried out for a sufficient period of time until NDA is obtained.

As the reaction proceeds, the molecular oxygen is absorbed and a large quantity of heat of the reaction is generated. Hence, it is usually unnecesary to heat the reactor externally during the oxidation reaction but rather it is necessary to remove the heat and maintain the reaction system at the predetermined reaction temperature. The heat can be easily removed by known mehods, for example by an internal heat removal procedure such as evaporation of the reaction solvent or the releasing of the blown gas, or by an external heat removing procedure such as cooling with a cooling medium such as water or water vapor, or by using both of these procedures.

When the starting material in the reaction system disappears and the termination of the reaction draws near, the absorption of molecular oxygen almost ceases apparently. Sometimes, at this stage, a reaction intermediate not completely converted into NDA is seen to exist. In such a case, the reaction mixture, as required, is further contacted with molecular oxygen (post-oxidation) to complete the oxidation reaction. This increases the yield of NDA, and at the same time, oxidatively decomposes unwanted by products and intermediates to increase the purity of the resulting NDA. The post-oxidation is carried out in the same oxidation reactor subsequently to the main oxidation reaction. Alternatively, after the main oxidation reaction, the reaction mixture is transferred to a separate vessel, and contacted with molecular oxygen for a predetermined period of time. The reaction pressure and temperature for the post-oxidation need not to be the same as in the main reaction, and may be higher or lower.

After the reaction, the isolation and recovery of NDA from the reaction product mixture, the purification of NDA, and the after-treatment of the mother liquor left after removal of NDA and its recycling and re-use may be carried out by conventional methods known, for example, in the production of NDA from other starting materials such as 2,6-dimethylnaphthalene or the production of terephthalic acid from p-xylene.

Essentially, the process of this invention can be carried out batchwise or continuously. However, as stated above the concentration of the starting DIPN or its oxidation derivative should be maintained low during the reaction, and the batch reaction is not always practical. Preferably, the oxidation is carried out continuously or by a semi-continuous method in which the starting material is added little by lttle to a solution of the catalyst either batchwise or continuously.

The catalyst may be added in a required amount to the reactor before the reaction is started. It may also be added continuously, batchwise or intermittently during the reaction.

After the reaction, the main product NDA is isolated from the reaction mixture. As required, water, TMA and other by-products are partly or substantially mostly from the remainder. The mother liquor is recycled to the oxidation reaction to enable the unreacted starting material, oxidation intermediates and the catalyst to be reused. At this time, water may be removed by evaporation, for example. TMA is removed, for example, by solid-liquid separation utilizing the formation of a water-insoluble salt with cobalt or manganese.

By the process of this invention, NDA can be obtained in a higher purity and a higher yield by any of the previously known processes, and high-quality NDA can be obtained from DIPN industrially at low costs.

The following Examples and Comparative Examples illustrate the present invention more specifically. In these examples, all parts are by weight.

## EXAMPLE 1

A titanium-lined pressure reactor equipped with a reflux condenser, a gas blowing tube, a discharge tube, a pump for continuous feeding of the starting material and a stirrer was charged with 150 parts of

propionic acid (HOPrn), 3.114 parts of cobalt acetate tetrahydrate [$Co(OAC)_2.4H_2O$], 3.064 parts of manganese acetate tetrahydrate [$Mn(OAc)_2.4H_2O$], 11.900 parts of potassium bromide and 9.814 parts of potassium acetate (KOAc). At a temperature of 200 °C and a pressure of 30 $kg/cm^2$-G, the compounds were stirred vigorously at a linear peripheral velocity of 100,000-12,000 m/hr, and 53.08 parts of 2,6-diisopropylnaphthalene (DIPN) was fed into the reactor continuously over the course of 4 hours, An excessive amount of compressed air was passed through the reactor to oxidize DIPN.

After feeding all DIPN, air was further passed for one hour through the reactor at 200 °C and 30 $kg/cm^2$-G to perform post-oxidation and complete the reaction. The reaction product was taken out, and a solid precipitate composed of 2,6-naphthalenedicarboxylic acid (NDA) was separated. The precipitate was washed and dried to give yellowish white fine crystals. Gas-chromatographic analysis showed that the fine crystals were 46.53 parts of NDA.

From the mother liquor left after separation of the precipitate, most of propionic acid was removed by distillation. The residue was heated with about 150 parts of water to dissolve inorgnaic salts of cobalt, manganese, bromine and potassium. After cooling, the solution was filtered to give a pale yellow brown precipitate. Gas-chromatographic analysis showed it to consist of 1.63 parts of NDA, 5.42 parts of TMA (trimellitic acid) and tiny amounts of intermediates and other by-products. Part of TMA was contained in the filtrate, and the above-indicated amount is the total amount of these.

Accordingly, the yield of NDA was 89.11 % and the yield of TMA was 10.32 % (both molecular percent based on the starting DIPN used; the same basis hereinafeter).

## EXAMPLES 2-12 AND COMPARATIVE EXAMPLE 1-7

The same reactor as used in Example 1 was charged with 150 parts of propionic acid, and as a catalyst, cobalt acetate, manganese acetate, potassium bromide and potassium acetate so that the amounts X, Y, Z and W (which are gram atoms in atomic percent of cobalt, manganese, bromine and potassium respectively per mole of the starting DIPN) were as indicated in Table 1 below. At a temperature of 200 °C and a pressure of 30 $kg/cm^2$-G, these materials were stirred at a linear peripheral velocity of 10,000 - 12,000 m/hr, and 53.08 parts of DIPN was added continuously over 4 hours. At the same time, an excessive amount of compressed air was brought into contact with the reaction mixture to perform oxidation reaction. Furthermore, the same post-oxidation as in Example 1 was carried out for 1 hour.

The reaction product was worked up and analyzed as in Example 1. The results are shown in Table 1.

Table 1

| | Amount of the catalyst (atomic % / DIPN) | | | | Yield of NDA (%) |
|---|---|---|---|---|---|
| | X | Y | Z | W | |
| CEX- 1 | 1.5 | 1.5 | 5 | 10 | 40.73 |
| CEX- 2 | 1.5 | 1.5 | 20 | 40 | 58.06 |
| CEX- 3 | 1.5 | 1.5 | 60 | 120 | 38.56 |
| CEX- 4 | 1.75 | 1.75 | 40 | 80 | 72.34 |
| CEX- 5 | 2.5 | 2.5 | 10 | 20 | 55.62 |
| EX- 2 | 2.5 | 2.5 | 40 | 80 | 81.00 |
| CEX- 6 | 2.77 | 2.77 | 110 | 220 | 56.41 |
| EX- 3 | 4 | 4 | 40 | 80 | 86.50 |
| CEX- 7 | 5 | 5 | 5 | 10 | 54.84 |
| EX- 4 | 5 | 5 | 15 | 30 | 84.80 |
| EX- 5 | 5 | 5 | 30 | 60 | 90.30 |
| EX- 1 | 5 | 5 | 40 | 80 | 89.11 |
| EX- 6 | 5 | 5 | 60 | 120 | 90.15 |
| EX- 7 | 10 | 10 | 20 | 40 | 94.33 |
| EX- 8 | 10 | 10 | 40 | 80 | 94.87 |
| EX- 9 | 15 | 15 | 20 | 40 | 95.58 |
| EX-10 | 15 | 15 | 40 | 80 | 94.47 |
| EX-11 | 20 | 20 | 5 | 10 | 90.45 |
| EX-12 | 20 | 20 | 40 | 80 | 94.23 |
| EX = Example; CEX = Comparative Example | | | | | |

EXAMPLES 13-17

The procedure of Examples 2 to 12 was repeated except that acetic acid or a mixture of acetic acid and propionic acid was used instead of propionic acid. The results are shown in Table 2.

Table 2

| | Amount of the catalyst (atomic % / DIPN) | | | | Solvent (parts) | | Yield of NDA (%) |
|---|---|---|---|---|---|---|---|
| | X | Y | Z | W | HOAc | HOPrn | |
| EX-13 | 5 | 5 | 30 | 60 | 150 | 0 | 81.50 |
| EX-14 | 5 | 5 | 40 | 80 | 75 | 25 | 87.75 |
| EX-15 | 5 | 5 | 40 | 80 | 75 | 75 | 88.07 |
| EX-16 | 5 | 5 | 40 | 80 | 66.7 | 33.3 | 92.50 |
| EX-17 | 10 | 10 | 40 | 80 | 75 | 75 | 94.25 |

EXAMPLES 18-20 AND COMPARATIVE EXAMPLE 8

The procedure of Examples 2 to 12 was repeated except that the proportions of cobalt and manganese were changed as indicated in Table 3. The results are shown in Table 3.

Table 3

| | Amount of the catalyst (atomic % / DIPN) | | | | Yield of NDA (%) |
|---|---|---|---|---|---|
| | X | Y | Z | W | |
| CEX-8 | 10 | 0 | 40 | 80 | 20.99 |
| EX-18 | 7.5 | 2.5 | 40 | 80 | 88.07 |
| EX-19 | 3 | 7 | 40 | 80 | 88.57 |
| EX-20 | 0 | 10 | 40 | 80 | 84.79 |

## EXAMPLES 21-22

The same reaction as in Example 1 was carried out at different temperatures and pressures.

Specifically, a titanium-lined pressure reactor equipped with a reflux condenser, a gas blowing tube, a discharge tube, a pump for continuous feeding of the starting material and a stirrer was charged with 150 parts of propionic acid, 3.114 parts of cobalt acetate tetrahydrate, 3.064 parts of manganese acetate tetrahydrate, 11.900 parts of potassiumk bromide and 9.814 parts of potassium acetate. With vigorous stirring at a linear peripheral velocity of 10,000-12,000 m/hr at 200 °C and 20 $kg/cm^2$-G (Example 21) or 180 °C and 15 $kg/cm^2$-G, 53.08 parts of DIPN was continuously fed over 4 hours. An excessive amount of compressed air was passed through the reactor to perform oxidation reaction.

After feeding all DIPN, the reaction mixture was further maintained at the same temperature and pressure, and passing of air was continued for 1 hour to perform post-oxidastion and complete the reaction.

The reaction product was worked up and analyzed as in Example 1.

The yield of NDA was 90.80 % in Example 21 and 87.47 % in Example 22.

## EXAMPLES 23-24 AND COMPARATIVE EXAMPLE 9

Example 1 was repeated except that the amount of the propionic acid solvent was changed to 300 parts (Example 23), 100 parts (Example 24) or 50 parts (Comparative Example 9).

The colors and yields of the resulting NDA were as follows:-

Example 23: pale ocher, 77.50 %
Example 24: slightly yellowish white, 92.88 %
Comparative Example 9: yelllow brown, 54.29 %

## COMPARATIVE EXAMPLES 10-11

These examples show that when X, Y and Z values are maintained proper but the Z/W ratio is outside the scope of the invention, the effect of the invention is not sufficiently produced.

Example 1 was repeated except that the proportion of potasium bromide was changed to 4.463 parts and the proportion of potassium acetate was changed to 35.577 parts (Z=15, W=160; Comparative Example 10), or the proportion of potassium bromide was changed to 5.950 and potassium acetate was not used (Z=20, W=20; Comparative Example 11).

In Comparative Example 10, the oxidation proceeded nearly in good condition to give pale ocher NDA. Its yield was only 74.68 % and lower than in Example 4.

In Comparative Example 11, the catalyst underwent deactivation soon after the start of the reaction, and the absorption of oxygen in the reaction system gradually decreased. In about 3 hours, the oxygen absorption became more unstable, and oxygen was obsorbed only intermittently. After the reaction, all the DIPN added disappeared, but a large amount of a black brown by-product of an unknown structure formed,

and yellow brown NDA was obtained only in a yield of 53.54 %.

## Claims

1. A process for producing 2,6-naphthalenedicarboxylic acid (NDA) which comprises oxidizing 2,6-diisopropylnaphthalene (DIPN) or its oxidation derivative with a gas containing molecular oxygen in a solvent containing at least 70 % by weight of at least one aliphatic monocarboxylic acid selected from the group consisting of acetic acid and propionic acid; wherein the oxidation is carried out

(a) using at least 1.5 parts by weight per part by weight of the entire reaction mixture composed of the material to be oxidized, its intermediates and product from these which are present in the oxidastion reaction system,

(b) using a catalyst comprising manganese, or a heavy metal mixture composed of cobalt and manganese with a manganese content of not more than 20 atomic percent, bromium and potassium in an amount satisfying the following expressions (i) to (v) simultaneously

$$\begin{array}{llll} (i) & (X + Y) \geq (100/Z + Z/20) \\ (ii) & (X + Y) \leq 50 \\ (iii) & Z \leq 60 \\ (iv) & Z < W \leq 8Z \\ (v) & W \leq 120 \end{array}$$

wherein X, Y, Z and W are the gram atoms, in atomic percent, of cobalt, manganese, bromine and potassium,
respectively, per mole of the starting 2,6-diisopropylnaphthalene or its oxidation derivative, and

(c) at a temperature in the range of 180 to 220 °C.

2. The process of claim 1 in which the amount of the catalyst simultansously satisfies the following expressions (i) to (v):

$$(i) \quad (X + Y) \geq (160/Z + Z/8)$$

$$\begin{array}{llll} (ii) & (X + Y) \leq 40 \\ (iii) & Z \leq 50 \\ (iv) & Z < W \leq 6Z \\ (v) & W \leq 100 \end{array}$$

3. The process of claim 1 in which the oxidation is carried out while adding the starting 2,6-diisopropylnaphthalene or its oxidation derivative continuously or intermittently at a rate of not more than 1 part by weight per 2 parts by weight of the solvent per hour.

4. The process of claim 1 in which the solvent is composed of 0 to 75 % by weight of acetic acid and 100 to 25 % by weight of propionic acid.